# EUROPEAN PATENT APPLICATION

(11) **EP 3 246 862 A1**
(43) Date of publication of application: **22.11.2017**
(21) Application number: 17170717.7
(22) Date of filing: 11.05.2017
(51) Int. Cl.: G06Q 10/08, G06Q 50/22

(54) **DISPENSING PROCESS OF MEDICAL PRODUCTS IN A HEALTHCARE FACILITY**

(30) Priority: 18.05.2016 IT UA20163573
(71) Applicant: PHARMATHEK S.r.L., 37136 Verona (IT); ESSEGI SOFTWARE S.r.L., 25131 Brescia (IT); SOL GmbH, 55276 Oppenheim (DE)
(72) Inventor: VON LIECHTENSTEIN, Alexander, FL-9497 TRIESENBERG (LI); Jens, WIEGLAND, D-67578 GIMBSHEIM (DE); VANNINI, Luca, I-37066 SOMMACAMPAGNA (Verona) (IT)
(74) Representative: Lunati & Mazzoni S.r.L.

(57) **Abstract**

There is provided a process (1) of dispensing medical products (2) in a healthcare facility comprising a related pharmacy, the medical products (2) being mainly intended for sale to the public and comprising at least one secondary packaging, the dispensing process (1) comprising: at least one step (10) of monitoring the stocks of medical products (2), at least one step (11) of supplying the related pharmacy with the medical products (2), at least one step (12) of storing the medical products (2) in the related pharmacy, at least one step (13) of releasing the medical products (2) in at least one ward of the healthcare facility, and at least one step (14) of sorting the medical products (2) to at least one destination patient (3) in the wards of the healthcare facility, wherein the storing step (12) comprises: a step (120) of singularizing the medical products (2), in which the secondary packages (21) are divided into a plurality of secondary packaging units (22), a step (121) of cataloging the secondary packaging units (22) in which the secondary packaging units (22) are inserted inside labeled packages (23), and wherein the storing step (12), the releasing step (13) and the sorting step (14) provide feedbacks to the monitoring step (10).

## Description

The present invention relates to a dispensing process of medical products, in a healthcare facility, of the type specified in the preamble of the first claim.

In particular, the invention relates to a process which provides for the optimization of the storage of medical products, e.g. such as tablets or other, supplying them to healthcare facilities correlated to a pharmacy, such as an internal pharmacy or an associated external pharmacy.

In medical context, and particularly with reference to hospital activities, medical products are of fundamental importance for curing and stabilizing the health condition of patents.

They are purchased by the healthcare facilities, in a number suited to the internal need of the facilities themselves, and undergo a cataloging and dispensing process which conveys such products to the end recipients, i.e. the patients.

The process as a whole mainly comprises purchasing the goods, storing and supplying the pharmacy, delivering to the hospital wards, storing and supplying the wards, providing to the patents and replenishing the pharmacy, which in turn supplies the ward again.

It further depends on various factors, such as in particular: requirements in standards or regulations, e.g. such as mandatory replenishment frequencies, economic indications, e.g. such as the amount of the budget allocated to medicaments or agreements with suppliers, and the specific requirements of the process, e.g. such as dispensing times and frequencies.

The process as a whole involves various hospital wards and professionals with specific requirements and different responsibilities. Such a complexity requires univocally defined processes and interfaces, capable of accurately reproducing the entire process.

Specifically, medical products are directly provided to patients with variable timing and procedures during their hospitalization in the facility and by operators, the nurses, who are in charge of managing and caring for the physiological state of many individuals, but who may also perform other tasks within the facility. Especially for the latter reason, the dispensing of medicaments to the patients must be optimized also from the procedural point of view, complying with the regulations in force, not only from the organizational point of view (univocity).

With reference to procedural aspects, before the administration, the healthcare operators must have a defined zone, within the room, for preparing the medicament, a dedicated resting surface, suitable clothing, the necessary for washing and/or disinfecting their hands, the drugs to be prepared (after checking correspondence between prescription and product) and all the technical information needed to ensure a correct and safe preparation of the prescribed drugs (drug information sheet or technical data sheet).

During preparation and administration, they must also fill in and apply the label onto the container of the drug. The label must not cover the identification elements of the product and must contain the following minimum information: full name of the patient, name of the drug, doses and amounts, administration route process, date and time of preparation and expiry date and time, if applicable (if administration is not immediate).

The described prior art as described comprises some major drawbacks.

Firstly, the supplying, storing and, especially, administrating process requires a long time to fully perform the operations listed above.

Furthermore, the healthcare operators must substantially respect what is commonly known as the 6R rule (R = Right, meaning correct) without any kind of error. This rule presupposes that the right activity is performed considering the right drug, the right dosage, the right route, the right time, the right patient and, finally, the right registration.

Another evident disadvantage is thus the complexity of the operations that the nurses must perform to complete their tasks as a whole and which implies, in many cases, an incorrect administration of the concerned medicaments. Indeed, the products used often do not respond to process needs, especially in reference to administration, because, for example, the packages of the products are not of the right size for storing in the ward or the necessary codes for implementing checks, allocations, checks and subsequent monitoring are missing, resulting in promoting the increase of the error percentage.

Such an error may concern one or more rules to be followed, but leads to severe consequences, especially if related to incorrect dosage of particularly invasive drugs.

The prior art is inconvenient because it is not rare for patients, for example, to have cognitive, visual and auditory impairments; it is therefore possible for the patients themselves, possibly provided with the entire package of the medicament, to take incorrect doses of the drug, with sometimes severe consequences for their health.

Indeed, it is not possible to resort to single and customized dosages unless they are previously or concurrently prepared by hospital personnel, thus resulting in an increase in terms of time and operative rapidity.

As previously mentioned, the medical products, e.g. such as common tablets, are dispensed by drug makers in conventional packages characterized by graphic or informative elements, which indicate the content, and which contain one or more heat-formed polymeric sheets, also named blisters, incorporating multiple doses therein.

The preliminary preparation, i.e. not concurrent to the visit to patients, of the correct dose is made impossible by the risk of environmental decay over time which elapses between the preparation of the single dose and the taking; the concurrent preparation is included in the disadvantages, mentioned above, in terms of procedure and times.

However, some solutions for the preliminary preparation of the single doses are present in the current art: reference is made, in this sense, to patent application US-A-3754374, FR-A-2965171, US-A-2009/0120042, US-A-2011202174, US-B-7971414.

Firstly, all the listed solutions have the disadvantage of only partially solving some drawbacks of the conventional prior art, because they are limited to only some aspects of the dispensing process whilst neglecting others, and furthermore they include the use of complex and complicated machinery (US-A-3754374, US-A-2009/0120042), the use of complicated computerized systems and of singularizing systems of the medical products, also in this case only partial, allowing only to create customizable blisters (US-A-2011/202174A1, US-B-7971414), or are not provided, when they allow actual singularization, with the function of being able to register the administration to the patient (FR-A-2965171), thus not constituting significant advantages for optimization of the entire process.

In all cases, the aforesaid techniques have the severe disadvantage of being not very economical, especially compared to the limited scope to which they are exclusively dedicated.

In this situation, it is the technical task underlying the present invention to devise a dispensing process of medical products capable of substantially rectifying the mentioned drawbacks.

In the scope of said technical task, it is an important object of the invention to obtain a dispensing process of medical products which is more rapid with respect to the prior art.

It is another important object of the invention to provide a process which considerably simplifies the management of the resources dedicated to the administration to patients which is more economical than the current technique. Therefore, as a consequence of the previously expressed object, it is a further object of the invention to reduce the procedural errors of nurses and to prevent patents from taking an incorrect drug dose.

Finally, it is the technical task underlining the present invention to create a process which allows to prepare single doses avoiding the decay deriving from the exposure of the medicinal substances to the environment.

The technical task and the specified objects are achieved by the technical solution expressed in appended claim 1. Examples of preferred embodiments are described in the dependent claims.

The features and advantages of the invention will be explained in the following detailed description of preferred embodiments of the invention, with reference to the accompanying drawings, in which:

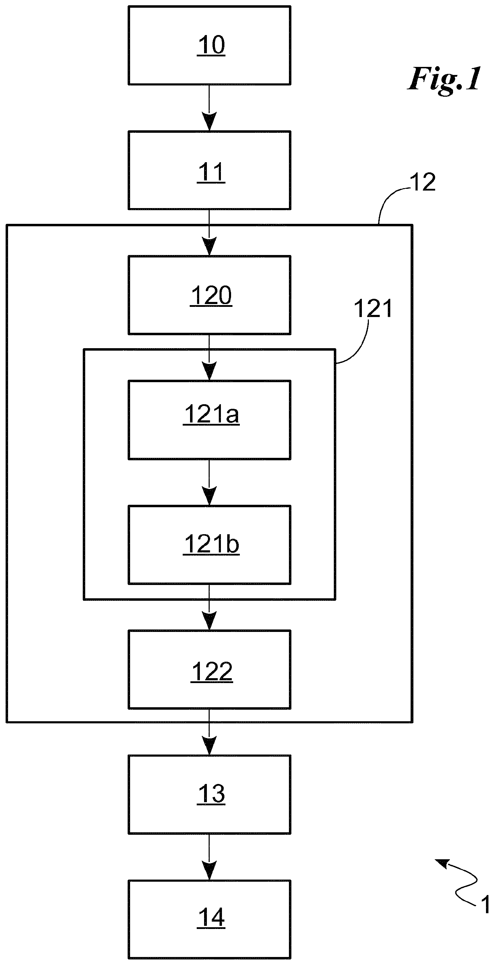

**Fig. 1** shows a diagram of the dispensing process;

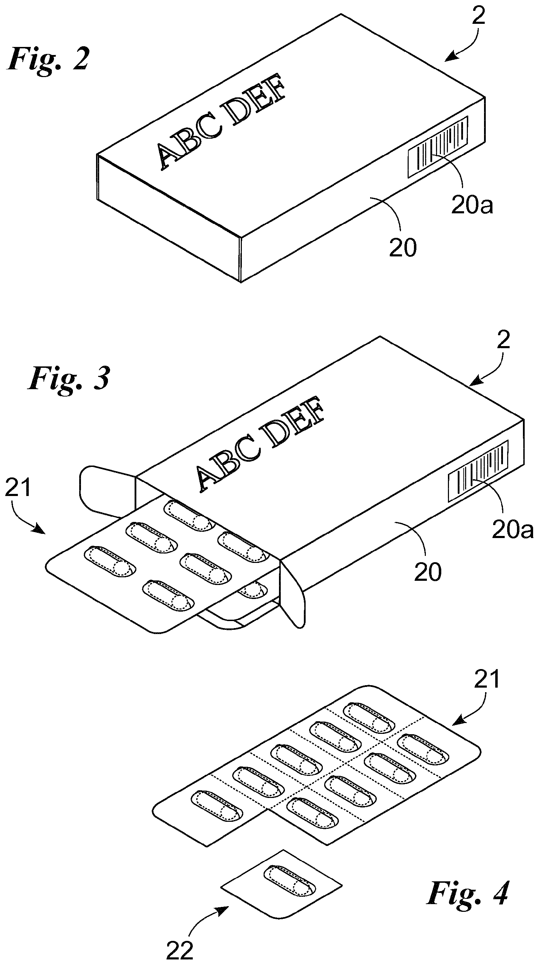

**Fig. 2** shows a generic medical product package;
**Fig. 3** shows the secondary packaging in the primary packaging of the medical product;
**Fig. 4** shows an example of singularization in a secondary packaging unit;

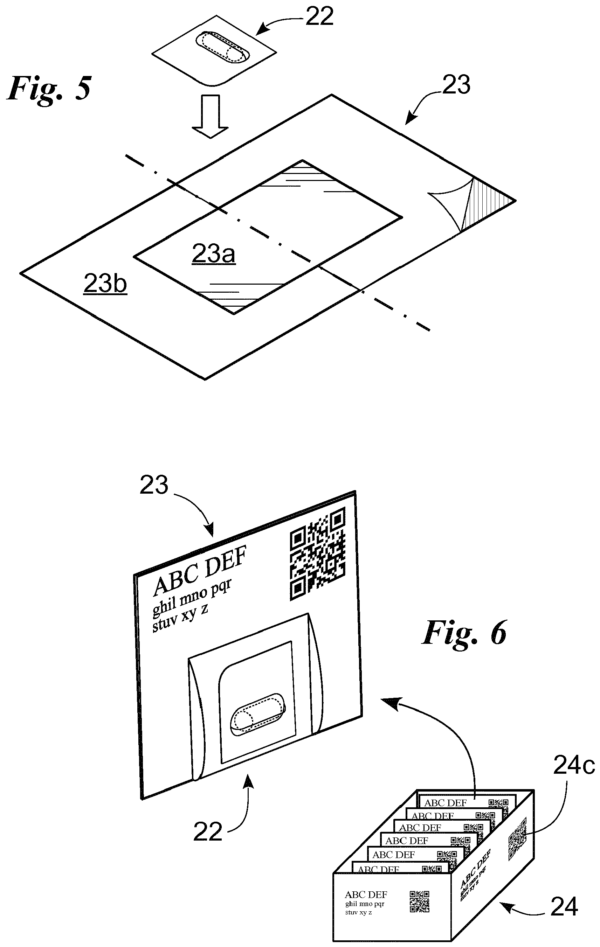

**Fig. 5** shows the introduction of a secondary packaging unit in the foldable sheet;
**Fig. 6** shows the labeled package and the storage container;

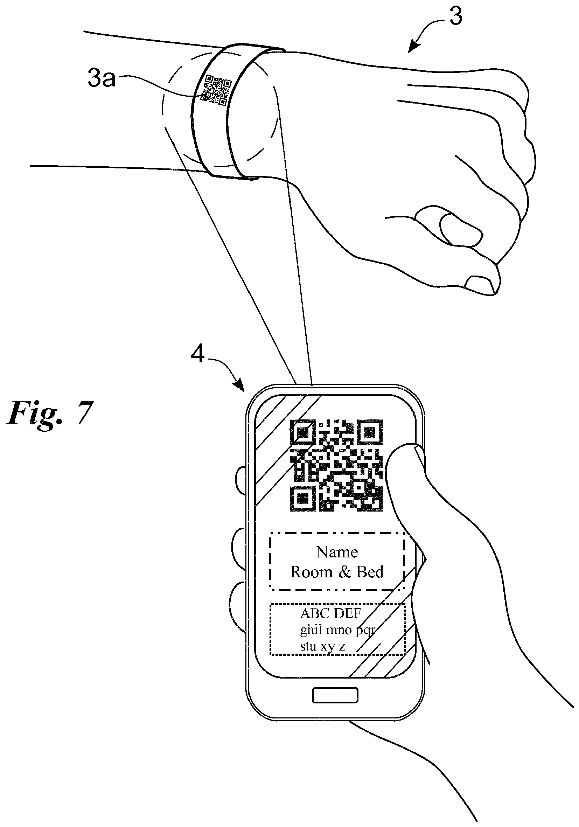

**Fig. 7** shows an example of patient data acquisition for administration; and

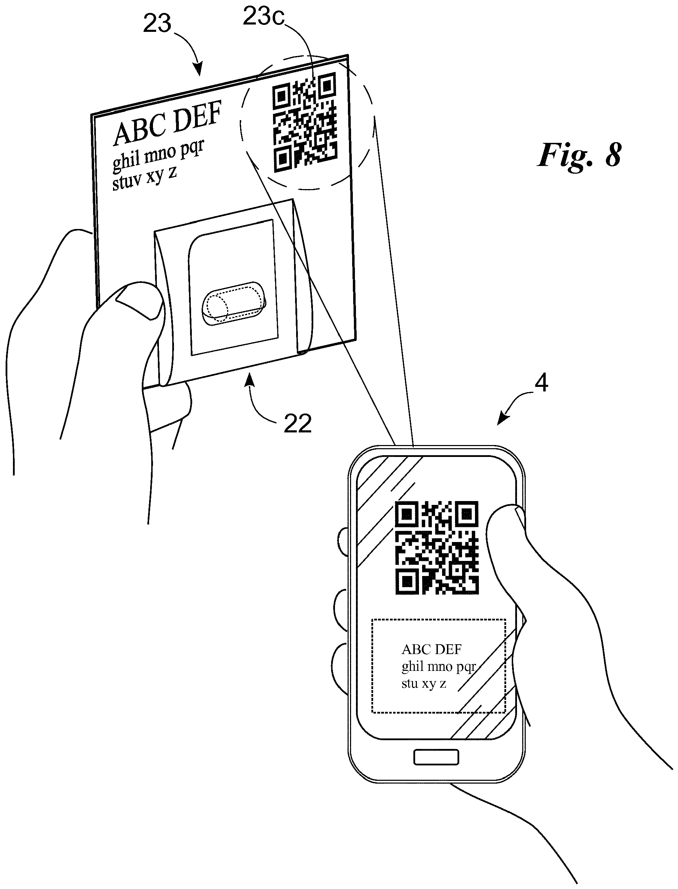

**Fig. 8** shows an example of registration and checking of the labeled package given to the patient.

In this document, when used, words such as "first", "second", "upper", "lower", "main" and "secondary" do not necessarily identify an order, a relationship priority or a relative position but may be simply used to distinguish different components more clearly.

With reference to the Figures, the dispensing process of medical products according to the invention is indicated by reference numeral **1** as a whole.

It is adapted to be implemented in a healthcare facility, in particular a hospital related to a pharmacy, such as an internal pharmacy or an associated external pharmacy, or a nursing home.

The healthcare facility may comprise a related pharmacy and at least one ward.

A pharmacy ID (e.g. a QR code or a barcode), which can be read by means of a specific decoder or reader, may be associated with the related pharmacy.

A ward ID (e.g. a QR code or a barcode), which can be read by means of a specific decoder or reader, may be associated with each ward.

The dispensing process of medical products is adapted to provide medical products **2,** e.g. tablets, pills, capsules, liquids and much more, to the patients of the healthcare facility.

Said medical products 2 are usually packed for selling to the public and for private users who store them in pharmacies open to the public, and not from pharmacies related to hospitals and the like.

The medical products 2 thus generally comprise a primary packaging **20,** such as a cardboard box or the like, and a secondary packaging **21,** generally inside the primary packaging, and for example consisting of blisters and the like. Each secondary packaging 21 usually contains only a plurality of single medical products 2. Said primary 20 and secondary 21 packaging is preferably optimized for selling to the public, as previously described. The primary packaging 20 thus contains important information for the sale, the preservation, the expiry of the medical product 2 and other information. This information is generally contained in the easily machine-readable sales code **20a,** such as a barcode or the like.

The process 1 of dispensing medical products 2 in healthcare facility comprises a plurality of labeled packages **23,** each comprising a product code **23c** identifying the labeled package 23.

The dispensing process 1 comprises storage containers **24** for the labeled packages 23 each of which comprising a container code **24c** of the container 24. The dispensing process 1 comprises, for example, a code reader 20a, 23c and appropriately 24c.

The dispensing process 1 comprises a computer comprising a medical product database associating at least part of information retrieved from said sales code 20a and at least, for example, either a drug ID or a ward ID with each product code 23c of the medical products 2, so as to know the amount of said medical product 2 in said related pharmacy and in said ward, in particular, the amount of medical product 2 can be identified on the basis of the product code number 23c associated with the pharmacy or ward.

The labeled packages 23, the storage containers 24, the reader and the computer and described below.

The dispensing procedure 1 preferably comprises one or more of the following steps: a step **10** of monitoring the medical product **2** stored in the related pharmacy, a step **11** of supplying the related pharmacy with the medical products 2, a step **12** of storing the medical products 2 in the related pharmacy, a step **13** of releasing the medical products in the healthcare facility wards, and a step **14** of sorting to the patients in the healthcare facility wards.

The steps and the information related to the medical products 2 in the related pharmacy may be contained in a medical product database. The latter associates at least part of the data contained in the sales code 20a, the amount of medical product 2 present in the related pharmacy, and the ward of the healthcare facility in which the medical product 2 is positioned with each medical product 2.

The medical product database may thus allow to know the amount of each medical product 2 present in the pharmacy and in each ward.

The medical product database is preferably managed by a computer, known *per se.*

The monitoring step 10 preferably comprises the use of the computer including the medical product database and capable of detecting the state of the stocks of the medical products 2 present in the pharmacy of the healthcare facility: such a system informs the pharmacy in case of lack of one or more medical products 2 so as to ensure a subsequent correct step of new supplying 11.

In this document, the lack of one or more medical products 2 may be defined when the amount of medical product 2 in the related pharmacy and/or in one or more wards is lower than a threshold (e.g. which can be determined on the basis of an estimate/average consumption and supply times) or if equal to 0.

The step 11 of supplying the pharmacy substantially comprises the simple purchasing of medical products 2, possibly arranged in their primary 20 and secondary 21 packaging, and thus in their packages intended for selling to the public.

The reader, if present, is preferably in data connection with the computer, and thus the product database.

The storing step 12 comprises the labeled packages 23 identifying a new packaging of the secondary packaging units 22 containing the medical products 2. The medical products 2 are adapted to be inserted, preferably singularly, in a labeled package 23, precisely in order to label the single medical products 2 and thus allow the registration of the stocks in the related pharmacy in the management software database.

Preferably, a labeled package 23 comprises specific sheets (preferably only one) folded and glued along the free edges. The mentioned sheets are advantageously simple to be made and used, but have all the features required in the context, e.g. of regular hospital activity: they preferably comprise a transparent central portion **23a** and a peripheral opaque portion **23b.**

Said sheets, once the secondary packaging units 22 are housed, are folded, e.g. symmetrically along the transversal axis, so as to either join or glue, or in all cases constrain, the edges of the sheets themselves and obtain the labeled packages 23.

The labeled packages 23, as the medical product database, may contain all the information referred to the medical products 2 inside, e.g.:
- name of the drug/medical product,
- name of the active principle,
- active principle dose for pharmaceutical form,
- batch,
- expiry date,
- information concerning the primary or secondary packaging 20, 21 from which the medical products 2 in the labeled package 23 were taken, preferably copied from the easily machine-readable sales code 20a,
- information including the number of labeled packages 23 included in a further container described below.

For this purpose, each labeled package 23 comprises the product code 23c, e.g. readable by means of a reader of known type, so as to access the aforesaid information contained in the pharmacy product software.

Therefore, in detail, when the product code 23c is read by a reader, information related to the medical product 2 contained in the labeled package 23 can be accessed, e.g. by means of a computer adapted to access the database.

Each product code 23c is preferably stored in the product database associated with a sales code 20a allowing the retrieval of said information.

The storing step 12 comprises storage containers 24 for the labeled packages 23. The storage containers 24 may include, in turn, a label which can be identified in a container code 24c appropriately similar to the product code 23c.

The container code 24c is adapted to associate a storage container 24 with the labeled packages 23 inserted therein, and thus with the medical products 2 contained in the storage container 24 itself.

The container code 24c may be stored in the product database associated with the product codes 23c of the labeled packages 23 inserted in the container 24, and thus to the one or more of the corresponding sales codes 20a.

The container code 24c may be associated with a ward ID in the product database.

The storing step 12 comprises, for example, a sales code reader 20a and said reader is appropriately of known type.

Said reader, if provided, may be adapted to read the codes 20a, 23c and 24c. The storage containers 24 are adapted to be positioned, and preferably are positioned, in automatic dispensers arranged in the related pharmacy.

Preferably, said automatic dispensers are of the type used in pharmacies open to the public, and for example described in patent application EP-B-2113472, paragraphs [0027]-[0084] and accompanying figures, and in patent application EP-B-2862817 [0022]-[0076] and accompanying figures, paragraphs which are incorporated herein for reference.

In order to be performed, the storing step 12 may appropriately include reading the sales code 20a by the reader; associating a product code number 23c not higher than (preferably equal to) the number of secondary packaging units 22 with the read sales code 20a and the pharmacy ID; and inserting the secondary packaging units 22 in the labeled packages 23, each comprising one of said product codes 23c. Preferably, in the storing step 12, one or more container codes 24c is associated with one or more product codes 23c and, in particular, containers are prepared 24 containing only the labeled packages 23 containing the product codes 23c associated with the container code 24c.

In particular, the storing step 12 preferably comprises one or more of the following steps: a step **120** of singularizing the medical products 2, a step **121** of cataloging the medical products 2 and, finally, a step **122** of packing the medical products.

In the singularizing step 120, the primary packaging 20 of the medical products 2 is opened, the secondary packaging 21, containing the medical products 2 is extracted.

The secondary packaging 21 is divided, in either manual or automated manner, into a plurality of secondary packaging units **22,** each including at least one single medical product 2, and preferably a single medical product 2.

For example, the secondary packaging 21 consisting of blisters is divided by cutting without damaging in any manner the external protection of the medical product 2 made in the secondary packaging 21. Therefore, the medical products 2 do not undergo variation of the external environmental conditions, e.g. light or humidity.

The subsequent cataloging step 121 preferably comprises a labeling step **121a** and a registering step **121b.**

Indeed, in the labeling step 121a, the reader may read the sales code 20a and send it to the computer. The computer, by virtue of the information present in the sales code 20a (of which the number of medical products 2 for package 20) associates a number of product codes 23c equal to the number of medical products 2 per package 20 with the sales code 20a; and then associates each of said product codes 23c with a single labeled package 23, e.g. by printing.

After having included the single secondary packaging units 22 in the labeled packages 23, they are registered in the registering step **121b** in the aforesaid mentioned software.

In this step, by exploiting the information contained in the sales code 20a, the computer updates the product database, increasing the number of those medical products 2 present in the related pharmacy and associating a product code 23c with each of them.

The packing step **122** comprises placing the labeled packages 23 of the medical products 2 in storage containers 24.

In particular, the computer may associate one or more product codes 23c with a container code 24c and may store such an association in the product database. The association may be performed on the basis of the medical products 2 lacking in the ward.

It is worth noting that product codes 23c associated with a single sales code 20a and/or with multiple sales codes 20a may be associated with container code 24c.

The operator or an automatic loading system may position the labeled packages 23 with the selected product code 23c in the container 24 with the correct container code 24c. During the releasing step 13, the computer preferably identifies the sales codes 20a of the medical products 2 lacking in a ward and, appropriately, identifies the number of units 22, and thus the necessary product codes 23a, in said medical product database; furthermore, for example, it may update the product database by varying the association of at least one product code 23c from the pharmacy ID with the ward ID (preferably on the basis of said unit number 2) of sales codes 20a of the lacking medical products 2; and controls the supply of the lacking medical products 2 in the ward.

The releasing step 13 comprises sending the storage containers 24 to the wards for which the medical products 2 are intended.

In this step, the storage containers 24 are correctly acquired by the wards, e.g. by associating each container code 24c with the ward ID for which it is intended.

In this phase, the storage containers 24 are, in turn, preserved in the common storage spaces in the wards themselves. Said transfers of medical products 2 are constantly registered in said medical product database.

In particular, the releasing step 13 may provide, if present, for the reader to read the container code 24c and send it to the computer, controlling the update of the product database. Indeed, upon reception of the container code 24c, the computer can update the product database deducting the products associated with the container code 24c from the medical products 2, i.e. the product codes 23c, present in the related pharmacy (i.e. associated with the pharmacy ID). Furthermore, the product database associates the container code 24 of the storage container 24 deposited in the ward itself with the ward, i.e. the ward ID.

At the end of the releasing step 13, the computer may impose a monitoring step 10.

The sorting step 14, in which the destination patients **3** are replenished with the correct medical products 2, occurs after the monitoring step 13.

Furthermore, the operations of providing or taking the single labeled packages 23, including the secondary packaging units 22 and the medical products 2, to the destination patient 3, are registered in the sorting step 14.

During the sorting step 14, the computer may update the product database deleting the association of the product codes 23c of the medical products 2 sorted with the ward ID and, in particular, by deleting said product codes 23c.

In order to allow such a registering, process 1 may comprise one or more electronic devices **4**, e.g. such as smartphones, communicating with the computer including the medical product database.

Information is used for registering said medical products 2, in particular the product code 23c, characterizing the single medical products 2 and a second identification code **3a** referred to the destination patient 3. Such an identification code 3a is preferably placed on a bracelet worn by the destination patents 3 or other.

Such a second identification code 3a, in detail, is preferably adapted to access the dose administration data referred to each patient and preferably inserted in a patent database.

For example, the latter patient database may be a database which may be compiled manually by healthcare operators so as to ensure the correct administration of medical products 2 to each patient 3.

The patent database can be implemented on one or more electronic devices 4 and/or the computer.

Preferably, a specific option is selected for said registering on the electronic device 4, and the patent identification code 3a, and possibly, also the product code 23c of the labeled package 23 are read by means of the same electronic device 4.

For example, each electronic device 4 may access the proper ward ID of the ward in which it is used so as to connect the product code 23c of the medical product 2 supplied to the patent to the ward ID or the container code 24c referred to the storage container 24 of the ward.

Thus, the sorting step 14 may substantially provide for the electronic device 4 to read the product code 23c and send it, appropriately together with the ward ID stored thereon, to the computer controlling the updating of the product database. Indeed, when such data are received, the computer updates the product database deducting from the medical products 2, i.e. from the product codes 23c, present in the ward, i.e. associated with ward ID sent by the electronic device.

If one or more medical products 2 are deemed lacking in the ward, the computer may impose the running of the releasing step 13 for at least said medical product 2 again.

In short, the process 1 of dispensing medical products 2 in a healthcare facility allows to obtain a regulation and an advantageous monitoring, in real time, of the medical products 2 involved in the process: the stocks of the pharmacy and the movements of the drugs involved in the releasing 13 and sorting 14 steps can be quantified.

By means of such data, reprocessed by the management software, it is thus possible to intervene in feedbacks on the stocks defining times and numerical needs concerning the supplying of the pharmacy and of the wards with the medical products 2.

For example, it is possible that only the ward and not the pharmacy need to be supplied: in such case, the management software can exclude the unnecessary step and allow a regular continuation of the subsequent steps of the procedure. The process 1 of dispensing medical products 2 in a healthcare facility according to the invention achieves important advantages.

Indeed, the process 1 of dispensing medical products 2 in the healthcare facility allows to perform the delivery functions of the single doses and storing of the delivery data more rapidly and with considerable simplicity of use. Such operations are carried out in real time and achieve high usefulness and simplicity, and low costs.

Such advantages are possible by virtue of the stock management software which allows to optimize the procedures and automate the step of releasing to the wards in real time.

It also allows to rapidly retrieve the information of the medical products 2 intended to the dose containers directly from the original package, allowing to substantially decrease the labeling sheet compilation times.

It is also useful to note that the process 1 of dispensing medical products 2 in healthcare facilities employs technologies, e.g. such as the smartphone acquisition system, which are included in daily use and represent a further simplification: in this sense, the learning time of the dispensing process of medical products 2 is shorter than more complex process.

The aforesaid advantageous features do not prevent achieving other important results from the operative point of view.

As mentioned, the "human factor" is a component to be taken into consideration when evaluating possible errors; indeed, clinical problems may occur deriving from incorrect dosing of the medicaments administrated to the patents by the healthcare operators. Such errors may be favorably minimized by the dispensing process of medical products 2 by means of alerts deriving from the application of reading identification code of the medical products 2 and patients 3, in case of incorrect administration. Finally, a further advantage of the dispensing process of medical products 2 is the limitation of possible dosing errors deriving from the patients themselves by virtue of the possibility of delivering single doses, the processing being simple e.g. manual, of the original secondary packaging.

Finally, the invention allows to use the medical products 2 with no waste, because the labeled packages 23 include a single medical product unit 2.

The invention is susceptible of variants included in the scope of the inventive concept defined by the claims. In such a scope, all the details can be replaced by equivalent elements and materials of any shape and size.

## Claims

1. A process (1) of dispensing medical products (2) in a healthcare facility comprising a related pharmacy and at least one ward,
- said medical products (2) being mainly intended for sale to the public and comprising an easily machine-readable sales code (20a), at least one secondary packaging (21) including a plurality of secondary packaging units (22), each including at least one single of said medical products (2);
- said dispensing process (1) being **characterized in that** it comprises:
- a computer comprising a medical product database associating at least part of said sales code (20a), the amount of said medical product (2) in said related pharmacy, and the ward of the healthcare facility in which said medical product (2) is positioned with each of said medical products (2),
- at least one monitoring step (10), wherein said computer identifies the medical products (2) lacking in said related pharmacy on the basis of said medical product database and controls the supplying of said lacking medical products (2),
- at least one step (11) of supplying said related pharmacy with said lacking medical products (2),
- at least one step (12) of storing said medical products (2) in said related pharmacy,
- comprising a step (120) of singularizing said medical products (2), wherein said secondary packages (21) are divided into a plurality of secondary packaging units (22),
- a step (121) of cataloging said secondary packaging units (22), wherein said secondary packaging units (22) are inserted in labeled packages (23) comprising at least one product code (23c), of easily machine-readable type, referred to said medical product (2) contained in the labeled package (23) and comprises information retrieved at least in part from said sales code (20a) related to the secondary packaging units (22) themselves,
- at least one step (13) of releasing said medical products (2) within said at least one ward of said healthcare facility, wherein said computer identifies the lacking medical products (2) in each of each of said at least one ward on the basis of said medical product database and controls the supplying of said lacking medical products (2) in said ward,
- at least one step (14) of sorting said medical products (2) to at least one destination patient (3) in said at least one ward of said healthcare facility,
- said storing step (12), said releasing step (13) and said sorting step (14) provide feedbacks to said computer, which updates said medical product database modifying said amount of said medical products (2) for said monitoring step (10).

2. A dispensing process (1) according to the preceding claim, wherein said cataloging step (121) comprises a step (121a) of labeling said labeled packages (23) and a step (121b) of registering said labeled packages (23).

3. A dispensing process (1) according to the preceding claim, wherein said labeled packages (23) are made by means of sheets folded and glued along the free edges.

4. A dispensing process (1) according to the preceding claim, wherein said destination patients (3) are identified by a second identification code (3a), and said identification code (3a) of said patient and said first product code (23c) of said labeled package (23) are read by means of an electronic device (4) in said sorting step (14).

5. A process (1) of dispensing medical products according to one or more of the preceding claims, wherein said first product code (23c) and said second identification code (3a) are QR codes.

6. A dispensing process (1) according to one or more of the preceding claims, wherein said storing step (12) comprises a packing step (122), wherein a plurality of said labeled packages (23) of said medical products (2) are arranged in storage containers (24).

7. A dispensing process (1) according to the preceding claim, wherein said storage containers (24) are inside automatic stores arranged in said related pharmacy.

8. A process (1) of dispensing medical products according to one or more of the preceding claims, wherein said monitoring step (10) comprises checking the stocks of said medical product (2) in said related pharmacy and in said wards.

9. A dispensing process of medical products in a healthcare facility (1) according to one or more of the preceding claims, wherein said steps of said dispensing process (1) are managed by means of a computer provided with management software.
